(19) Europäisches Patentamt / European Patent Office / Office européen des brevets

(11) **EP 4 481 695 A1**

(12) **EUROPEAN PATENT APPLICATION**

(43) Date of publication:
25.12.2024 Bulletin 2024/52

(21) Application number: 24182106.5

(22) Date of filing: 13.06.2024

(51) International Patent Classification (IPC):
*G06V 10/764* (2022.01)   *A61B 5/11* (2006.01)
*G06T 7/73* (2017.01)   *G06V 40/10* (2022.01)
*G06V 40/20* (2022.01)   *G08B 21/04* (2006.01)

(52) Cooperative Patent Classification (CPC):
**G06V 40/23; A61B 5/1116; A61B 5/1128;
G06T 7/73; G06V 10/764; G06V 40/103;**
G08B 21/043; G08B 21/0476

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 21.06.2023 JP 2023101409

(71) Applicant: **CANON KABUSHIKI KAISHA**
**Tokyo 146-8501 (JP)**

(72) Inventor: **TOJO, Hiroshi**
**Tokyo, 146-8501 (JP)**

(74) Representative: **Canon Europe Limited**
**European Intellectual Property Group**
**4 Roundwood Avenue**
**Stockley Park**
**Uxbridge UB11 1AF (GB)**

(54) **VIDEO IMAGE PROCESSING APPARATUS, POSTURE DETERMINATION METHOD, AND PROGRAM**

(57) A video image processing apparatus includes acquisition means (201) configured to acquire a video image, position determination means (202, 203) configured to determine a plurality of specific positions of a person in the video image acquired by the acquisition means (201), gravitational direction determination means (206) configured to determine a gravitational direction in at least a part of an area in the video image acquired by the acquisition means (201), and posture determination means (204, 207) configured to determine whether the person is in an unstable posture based on a determination result of the plurality of specific positions of the person by the position determination means (202, 203) and a determination result of the gravitational direction by the gravitational direction determination means (206).

**FIG.2**

**Description**

BACKGROUND OF THE INVENTION

Field of the Invention

The present invention relates to a determination method of an action of a person.

Description of the Related Art

Recently, a technique for detecting an action of a person from a video image of a monitoring camera has been proposed, and applications thereof to behavioral analysis of customers at a store or monitoring of patient behaviors in a hospital have been expanding. Further, a technique for analyzing an action of a person by estimating joint positions of the person in a video image has been proposed. However, it is difficult to detect an action of a person in a case where a posture looks the same as a posture taken in a different action, just from the person's joint positions. Japanese Patent Application Laid-Open Publication No. 2022-21940 discusses a method of detecting an object (e.g., chair) in the person's surroundings, and distinguishing, for example, between actions of "a person doing squats" and "a person sitting on a chair", based on a relationship between the target person's joint positions and the object's position.

However, according to the method discussed in Japanese Patent Application Laid-Open Publication No. 2022-21940, it is difficult to distinguish between an action of "a person falling on the person's bottom" and an action of "a person doing squats". Further, according to the method discussed in Japanese Patent Application Laid-Open Publication No. 2022-21940, it is also difficult to distinguish between an action of "a person standing in a passage" and an action of "a person lying down with the person's head pointed toward the back in an image capturing direction of a camera". As described above, there are cases where a plurality of actions not dependent on object positions cannot be distinguished based on the conventional art.

In consideration of the above-described issue, the present invention is directed to a method capable of distinguishing between person's actions accurately.

SUMMARY OF THE INVENTION

According to a first aspect of the present invention, there is provided a video image processing apparatus as specified in claims 1 to 11. According to a second aspect of the present invention, there is provided a posture determination method as specified in claims 12 to 14. According to a third aspect of the present invention, there is provided a computer program as specified in claim 15.

Further features of the present invention will become apparent from the following description of embodiments with reference to the attached drawings.

BRIEF DESCRIPTION OF THE DRAWINGS

Fig. 1 is a block diagram illustrating a hardware configuration example of a video image processing apparatus.
Fig. 2 is a block diagram illustrating a functional configuration of the video image processing apparatus.
Fig. 3 is a diagram illustrating an example of a skeletal frame determination result.
Fig. 4 is a flowchart illustrating a determination processing flow of a gravitational direction.
Fig. 5 is a diagram illustrating an example of a gravity vector field.
Fig. 6 is a diagram illustrating an example of vector selection to be used for a determination of the gravity vector field.
Fig. 7 is a diagram illustrating an example of a gravity vector field.
Fig. 8 is a diagram illustrating an example of a user interface.
Fig. 9 is a flowchart illustrating a processing flow of the video image processing apparatus.
Fig. 10 is a diagram illustrating an example of an unstable posture determination in a standing posture.
Fig. 11 is a diagram illustrating an example of an unstable posture determination in a sitting posture.
Fig. 12 is a diagram illustrating an example of an unstable posture determination in a case where a person has a body support instrument.
Fig. 13 is a block diagram illustrating a functional configuration of a video image processing apparatus.
Fig. 14 is a flowchart illustrating a processing flow of the video image processing apparatus.
Fig. 15 is a diagram illustrating examples of graphs of temporal changes of unstableness degrees.

DESCRIPTION OF THE EMBODIMENTS

Hereinbelow, embodiments of the present invention will be described with reference to the attached drawings. Note that the following embodiments are merely examples, and not intended to limit the scope of the present invention. In the attached drawings, the same or similar components are assigned the same reference numerals, and redundant descriptions thereof are omitted. In the embodiments, descriptions are given on an assumption that an "action" is identified by one posture or a plurality of consecutive postures. In other words, in the embodiments, a single posture can be considered as one action.

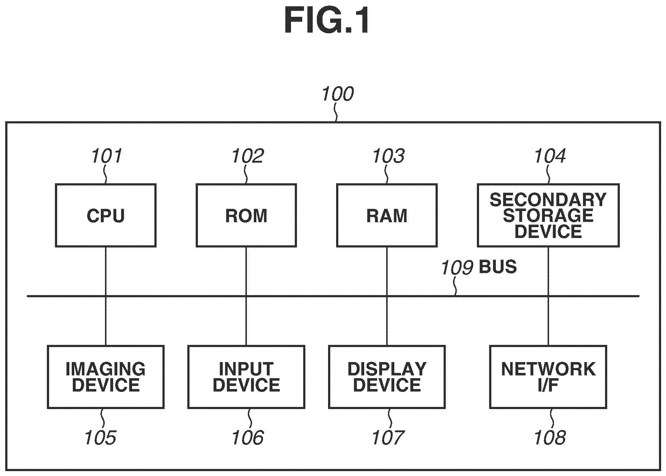

Fig. 1 is a block diagram illustrating a hardware configuration of a video image processing apparatus 100 according to an embodiment. The video image processing apparatus 100 according to the present embodiment includes a central processing unit (CPU) 101, a read only memory (ROM) 102, a random access memory (RAM) 103, a secondary storage device 104, an imaging device 105, an input device 106, a display device 107, a network interface (I/F) 108, and a bus 109.

The CPU 101 is a processor that executes instructions according to programs stored in the ROM 102 or the RAM 103. The ROM 102 is a non-volatile memory storing programs for executing processing relating to flowcharts described below, and programs and data required for other controls. The RAM 103 is a volatile memory for temporarily storing video/image data or a pattern determination result.

The secondary storage device 104 is a rewritable secondary storage device, such as a hard disk drive and a flash memory. Various kinds of information stored in the secondary storage device 104 are transferred to the RAM 103, and the CPU 101 can execute the programs according to the present embodiment. The imaging device 105 includes an imaging lens, an imaging sensor such as a charge-coupled device (CCD) sensor and a complementary metal-oxide semiconductor (CMOS) sensor, and a video image signal processing unit.

The input device 106 is a device for receiving an input from a user and is, for example, a keyboard and a mouse.

The display device 107 is a device for displaying a processing result or the like to a user, and is, for example, a liquid crystal display. The network I/F 108 is a modem or a local area network (LAN) for connecting to a network, such as the Internet and an intranet. The bus 109 is an internal bus for performing data input and output between the above-described hardware components by connecting them.

A determination method of an action and a posture in a first embodiment will be described in detail with reference to the drawings.

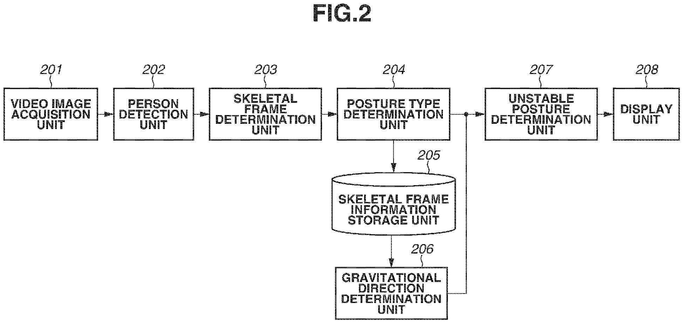

Fig. 2 is a block diagram illustrating a functional configuration of the video image processing apparatus 100. The video image processing apparatus 100 according to the present embodiment determines whether a person identified from a video image is in an unstable posture. The video image processing apparatus 100 includes a video image acquisition unit 201, a person detection unit 202, a skeletal frame determination unit 203, a posture type determination unit 204, a skeletal frame information storage unit 205, a gravitational direction determination unit 206, an unstable posture determination unit 207, and a display unit 208.

The video image acquisition unit 201 acquires a video image using the imaging device 105. In the present embodiment, the description is given focusing on an example in which the video image processing apparatus 100 includes the imaging device 105, but the video image processing apparatus 100 may not include the imaging device 105. In this case, the video image acquisition unit 201 of the video image processing apparatus 100 acquires a video image via a wired or wireless communication medium.

The person detection unit 202 detects an area of a person from the video image acquired by the video image acquisition unit 201. The person detection unit 202 according to the present embodiment detects an entire body area of a person.

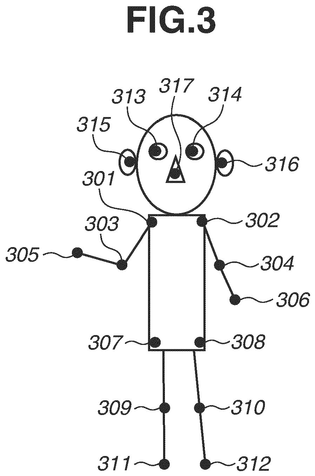

The skeletal frame determination unit 203 executes position determination processing for determining specific positions of a person from the entire body area detected by the person detection unit 202. The specific positions according to the present embodiment are illustrated in Fig. 3.

As illustrated in Fig. 3, the specific positions according to the present embodiment are shoulders 301 and 302, elbows 303 and 304, wrists 305 and 306, hips 307 and 308, knees 309 and 310, ankles 311 and 312, eyes 313 and 314, a nose 317, and ears 315 and 316.

In other words, the specific positions according to the present embodiment include positions of person's joints and organs.

The posture type determination unit 204 determines a type of posture of a person (e.g., standing posture or sitting posture) based on the specific positions determined (estimated) by the skeletal frame determination unit 203. The skeletal frame information storage unit 205 stores skeletal frame information in which information about the specific positions determined (estimated) by the skeletal frame determination unit 203 is associated with the type of posture determined by the posture type determination unit 204. The skeletal frame information storage unit 205 is implemented by the RAM 103 or the secondary storage device 104.

The gravitational direction determination unit 206 executes gravitation determination processing for determining a gravitational direction in at least a part of an area in the video image, based on the skeletal frame information stored in the skeletal frame information storage unit 205. A determination (estimation) method of the gravitational direction by the gravitational direction determination unit 206 will be described below.

The unstable posture determination unit 207 determines whether a person's posture is unstable, based on the gravitational direction determined by the gravitational direction determination unit 206 and the skeletal frame information stored in the skeletal frame information storage unit 205. The display unit 208 displays a determination result by the unstable posture determination unit 207. The display unit 208 is implemented by the display device 107.

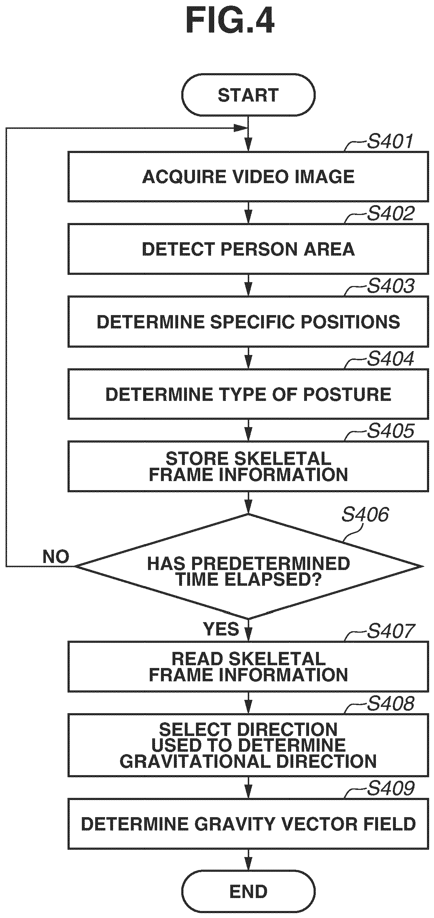

Next, details of processing performed by the video image processing apparatus 100 according to the first embodiment will be described. Fig. 4 is a flowchart illustrating processing executed by the video image processing apparatus 100 according to the present embodiment to determine (estimate) the gravitational direction. Each operation illustrated in Fig. 4 is implemented by the CPU 101 of the video image processing apparatus 100 reading a required program from the ROM 102 into the RAM 103, and executing the program. Further, the processing in Fig. 4 starts when an instruction to start a gravitational direction determination is issued by a user. However, for example, the processing in Fig. 4 may be started automatically when a predetermined condition is satisfied.

When the processing in Fig. 4 is started, in step S401, the video image acquisition unit 201 acquires a video image captured in a store.

Further, in step S401, the video image acquisition unit 201 associates time information (time stamp or frame identification (ID)) with each of image frames constituting the video image.

In step S402, the person detection unit 202 detects an area of a person (person area) from a frame image. The person detection unit 202 according to the present embodiment detects an entire body area including an entire body of a person as the person area. Examples of a method for the person detection unit 202 to detect a person area include a method of using convolutional neural network (CNN), as discussed in "J. Redmon "You Only Look Once: Unified, Real-Time Object Detection", CVPR2015". However, the method is not limited to this method, and any other methods may be used as long as the person area can be detected.

In the present embodiment, the person area is rectangular, and the position of the person area is indicated by x coordinates and y coordinates of two points that are one at an upper left position and one at a lower right position in a coordinate system with an upper left position of the frame image being an origin point. Further, time information of a frame image is assigned to each person area.

In step S403, the skeletal frame determination unit 203 determines the specific positions of a person with regard to all the person areas in the frame image. Then, the skeletal frame determination unit 203 generates information (joint point list) listing the specific positions for each person area. Examples of a skeletal frame determination (estimation) method include a method of estimating coordinates of each joint point using a CNN, as discussed in "Z. Cao, "OpenPose: Realtime Multi-Person 2D Pose Estimation using Part Affinity Fields", CVPR2018", and obtaining reliability thereof. However, it is not limited to this method, and any other methods may be used as long as the coordinates of the specific positions can be determined.

In the present embodiment, the joint point list is generated for each person area included in a frame image. Further, each joint point list is a list in which time information of the frame image, coordinates of all the specific positions of a person, and reliabilities are arranged in a specific order.

In the present embodiment, the description is given focusing on a case where the skeletal frame determination is performed on each person area detected from the entire frame image, but the skeletal frame determination may be performed on the entire frame image, and the skeletal frame determination may be organized for each person based on relationships between the specific positions.

In step S404, the posture type determination unit 204 determines a type of posture (e.g., standing posture or sitting posture) of the person based on the above-described joint point list. Examples of a method for determining the type of posture include a method of training a CNN or a multilayer perceptron (MLP) as a class identification problem, using a large amount of joint point lists prepared for each type of posture as training data. With this method, among likelihoods obtained for each type (class) of posture, the type of posture corresponding to the maximum likelihood is employed as the type of posture of the person.

In step S405, the skeletal frame information storage unit 205 stores a list in which posture type IDs are added to the joint point list as the skeletal frame information. A posture type ID is an ID set in advance for each type of posture, for example, 1 for a standing posture, and 2 for a sitting posture.

The video image processing apparatus 100 according to the present embodiment determines whether a predetermined time has elapsed. In a case where the predetermined time has elapsed (YES in step S406), the processing proceeds to step S407. On the other hand, in a case where the predetermined time has not elapsed (NO in step S406), the processing returns to step S401 to repeat the processing from steps S401 to S405 until the predetermined time elapses. It is assumed that persons appear at various positions in an imaging range while the predetermined time elapses, and the gravitational direction at each position in the imaging range can be acquired based on information acquired during the predetermined time. However, a condition for repeating steps from steps S401 to S405 is not limited to the predetermined time. For example, a condition in which the number of persons detected in a video image reaches a predetermined number, or a condition in which persons are detected at all positions in the frame image may be another condition.

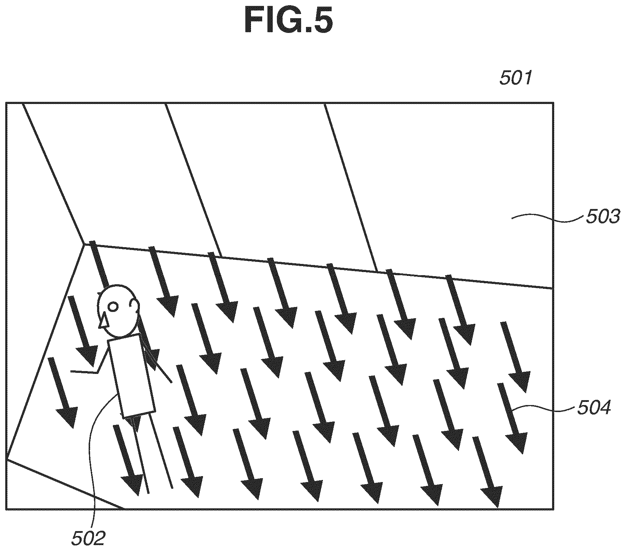

Trough the processing in steps S407 to S409, the gravitational direction is determined (estimated) in at least a part of the area in the video image. The video image processing apparatus 100 according to the present embodiment indicates a direction in which a gravitational force acts at each position on a floor surface or a ground surface in the video image using a unit vector. Fig. 5 illustrates an example thereof. A screen 501 illustrates an entire image, and a person 502 is standing in a room. A floor surface 503 is covered with arrows indicating gravitational directions represented by an arrow 504. In the present embodiment, the gravitational direction is associated with each of divided areas obtained by dividing a specific area, which is the floor surface or the ground surface in the image frame, into areas each with a predetermined size (10 pixels x 10 pixels in vertical and horizontal directions), and a divided area associated with one gravitational direction is referred to as a gravity vector field. However, the dividing method and the size of division are not limited to the above-described method and size.

In step S407, the gravitational direction determination unit 206 reads the skeletal frame information corresponding to a predetermined time period from the skeletal frame information storage unit 205. In step S408, the gravitational direction determination unit 206 selects a direction used to determine the gravitational direction from the joint point list included in the skeletal frame information.

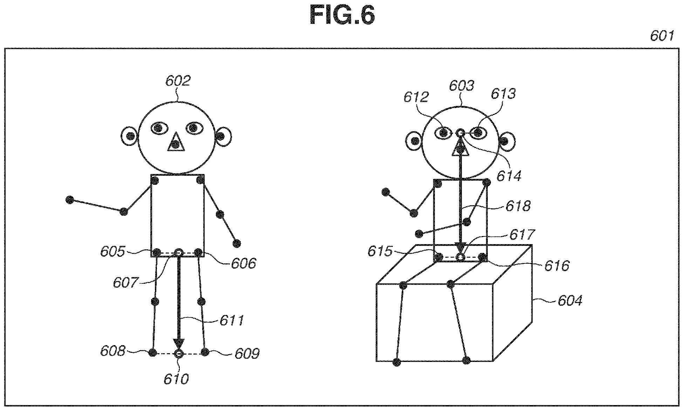

Details of the processing performed in step S408 will be described with reference to Fig. 6. Fig. 6 illustrates an example of an image 601 including a person 602 in a standing posture and a person 603 in a sitting posture. Black dots in each person indicate the specific positions illustrated in Fig. 3. The person 603 is sitting on a stool 604. A description will be given of processing of selecting a direction used to determine the gravitational direction for each of the two types of postures, a standing posture and a sitting posture.

The gravitational direction determination unit 206 selects a direction 611, which is from a midpoint 607 between hips 605 and 606 to a midpoint 610 between ankles 608 and 609 for the person 602 in a standing posture (posture type ID = 1). In the case of the standing posture, the person's head and upper part of the body may be tilted, but positions of the hips 605 and 606, and the ankles 608 and 609 usually take stable positions to support the person's body and to achieve a balance with respect to the gravitational direction. For this reason, the gravitational direction determination unit 206 according to the present embodiment selects the direction 611, which is from the midpoint 607 between the hips 605 and 606 to the midpoint 610 between the ankles 608 and 609. However, such a method is merely an example, and it is not limited thereto as long as the gravitational direction in a standing posture can be acquired accurately.

On the other hand, the gravitational direction determination unit 206 selects a direction 618, which is from a midpoint 614 between eyes 612 and 613 to a midpoint 617 between hips 615 and 616 for the person 603 in a sitting posture (posture type ID = 2). In the case of the sitting posture, since the person 603 supports the upper part of the body with the hips 615 and 616 to achieve a balance with respect to the gravitational direction, the person's hips 615 and 616 usually take stable positions. For this reason, the gravitational direction determination unit 206 according to the present embodiment selects the direction 618, which is from the midpoint 614 between the eyes 612 and 613 to the midpoint 617 between the hips 615 and 616. As described above, the gravitational direction determination unit 206 according to the present embodiment determines (estimates) the gravitational direction using a different method depending on whether the person is in a standing posture or a sitting posture. However, the method of selecting the gravitational direction of the person 603 in a sitting posture is not limited thereto. For example, instead of the midpoint 614 between the eyes 612 and 613, a center position between the eyes 612 and 613, and a nose may be used. Further, instead of the midpoint 617 between the hips 615 and 616, a center position between the hips 615 and 616 and both knees may be used. While the person's upper part of the body tends to be tilted in a sitting posture, by using a sufficient number of vectors, the gravitational direction can be highly accurately obtained.

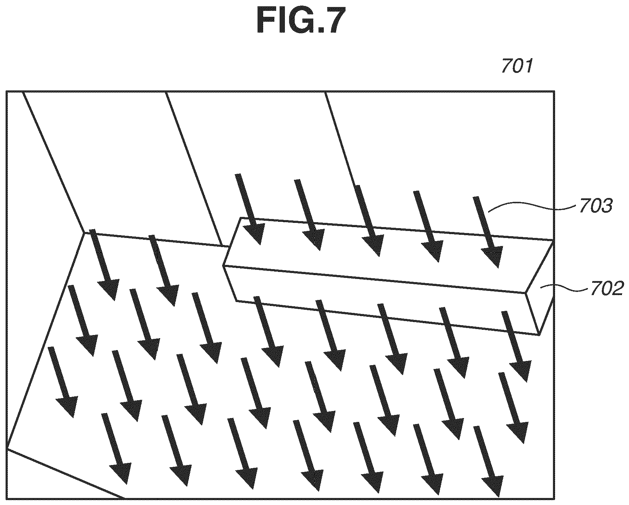

With the above-described method, in step S408, the gravitational direction determination unit 206 determines the gravitational direction based on the type of posture of the person. In this way, as illustrated in Fig. 7, in a case where a bench 702 is placed in a screen 701, it is possible to determine gravitational directions 703 not only on the floor surface but also on a bench surface.

In step S409, the gravitational direction determination unit 206 determines a gravity vector field from the gravitational direction acquired in step S408. As described above, in the present embodiment, the gravitational direction is associated with each of the divided areas obtained by dividing the specific area (e.g., floor surface, ground surface, and stool (bench) surface) in the image frame into the areas each with the predetermined size (10 pixels x 10 pixels in vertical and horizontal directions). More specifically, the gravitational direction determination unit 206 determines an average of all gravitational directions in a predetermined range from each divided area as the gravitational direction of the divided area.

The gravitational direction determination unit 206 according to the present embodiment determines the gravitational direction based on organ points corresponding to the type of posture of a person, but it is not limited thereto. For example, there is a method of determining depth information and semantic information in each pixel from an image using a vision transformer (ViT), as discussed in "Rene Ranftl, et al. "Vision Transformers for Dense Prediction" arXiv: 2103.13413". In this method, the gravitational direction with respect to a floor surface can be obtained from the relationship between the determined floor surface and the wall.

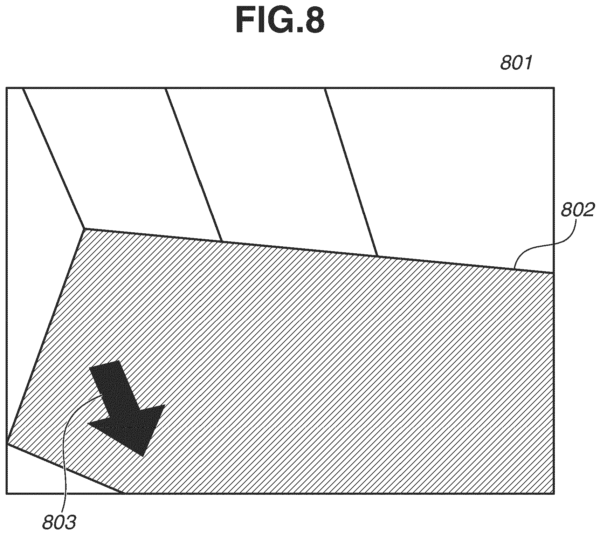

It is also possible for a user to input the gravitational direction via the input device 106. Fig. 8 illustrates an example of a user interface. A screen 801 illustrates an entire screen, and a user can designate a floor surface by operating a mouse or the like. A shaded area 802 is an example of the designated floor surface. The user can set the gravitational direction at each position on the floor surface by designating a position on the screen 801 with a mouse and inputting an angle using a keyboard, for example, to input a vector symbol 803 serving as a guide while visually confirming the inclination of the vector symbol 803.

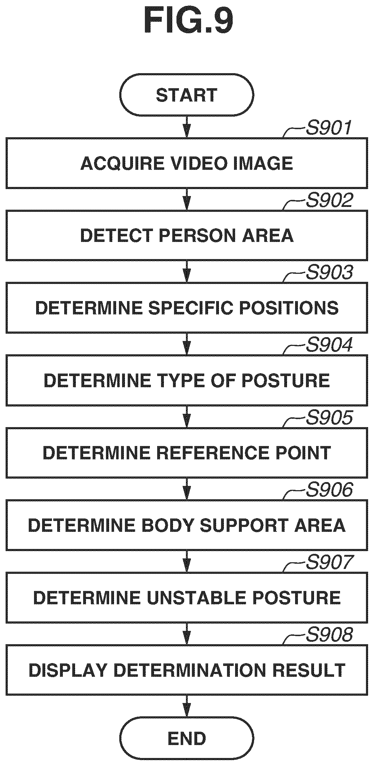

Next, details of processing when the video image processing apparatus 100 according to the first embodiment is in operation will be described. Fig. 9 is a flowchart illustrating processing executed by the video image processing apparatus 100 according to the present embodiment to determine (estimate) a person's posture. The processing in each step illustrated in Fig. 9 is implemented by the CPU 101 of the video image processing apparatus 100 reading a necessary program from the ROM 102 into the RAM 103 and executing the program. Further, the processing in Fig. 9 starts when a user issues an instruction to execute a posture determination. However, for example, the processing in Fig. 9 may be started automatically when a predetermined condition is satisfied.

In step S901, the video image acquisition unit 201 acquires a video image, as in step S401. In step S902, the person detection unit 202 detects an area of a person (person area) from a frame image constituting the video image, as in step S402. In step S903, the skeletal frame determination unit 203 determines specific positions (positions of joints or the like) in a frame image, as in step S403. In step S904, the posture type determination unit 204 determines a type of posture (e.g., standing posture or sitting posture) of the person based on the determination result of the person's specific positions, as in step S404.

In step S905, the unstable posture determination unit 207 determines a reference point used to determine whether the person's posture is unstable. In step S906, the unstable posture determination unit 207 determines a person's body support area.

In the present embodiment, the reference point is a specific position selected based on the type of posture of the person, and the unstable posture determination unit 207 determines whether the specific position is supported. Further, in the present embodiment, the person's body support area is an area used to determine whether the reference point is supported, and determined based on the person's posture. The unstable posture determination unit 207 according to the present embodiment determines the reference point and the person's body support area based on the specific positions corresponding to the type of posture of the person by a method similar to the method described in step S408.

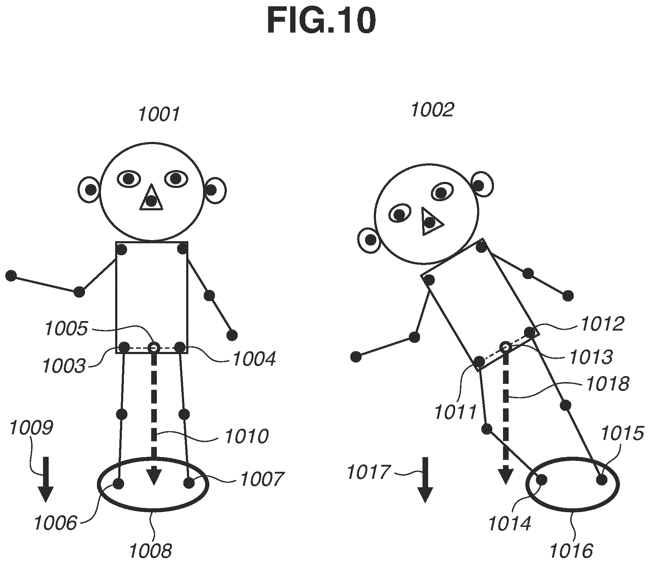

Fig. 10 illustrates a state where, of two persons determined to be in a standing posture based on the specific positions, a person 1001 is actually standing, and another person 1002 is actually about to fall down. For the persons 1001 and 1002 determined to be in a standing posture, the unstable posture determination unit 207 according to the present embodiment determines a midpoint between hips 1003 and 1004 as a reference point 1005 and a midpoint between hips 1011 and 1012 as a reference point 1013, respectively. As described above, it is because, in the case of a standing posture, the hips and the ankles usually serve as stable positions with respect to the gravitational direction, compared with the head and the shoulders. Further, the unstable posture determination unit 207 according to the present embodiment respectively determines an area 1008 including ankles 1006 and 1007 as a person's body support area and an area 1016 including ankles 1014 and 1015 as a person's body support area for the persons 1001 and 1002 determined to be in a standing posture. The person's body support area according to the present embodiment is a circle with the center at a midpoint between the ankles and with the diameter that is a distance obtained by adding a predetermined rate of margin to a distance between the ankles.

The determination method of the reference point and the person's body support area is not limited to the above-described method. For example, the reference point may be determined by including other joint points, such as shoulders, in addition to the hips. Further, the person's body support area may be determined by including other joint points, such as knees, in addition to the ankles. The person's body support area does not necessarily have to be a circle, and may be an ellipse or a rectangle.

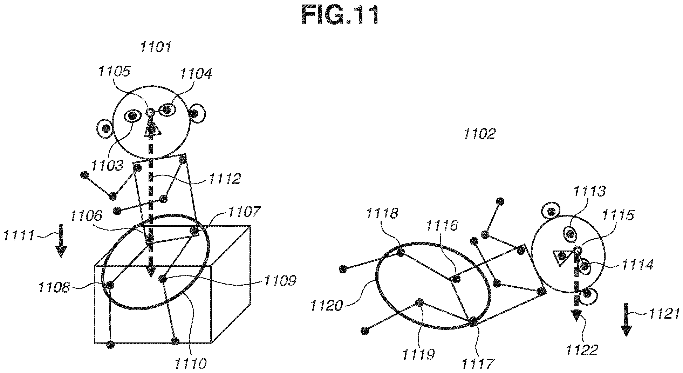

Fig. 11 illustrates a state where, of two persons determined to be in a sitting posture based on the specific positions, a person 1101 is actually sitting, and another person 1102 has actually fell backward. For the persons 1101 and 1102 determined to be in a sitting posture, the unstable posture determination unit 207 according to the present embodiment determines a midpoint between eyes 1103 and 1104 as a reference point 1105 and a midpoint between eyes 1113 and 1114 as a reference point 1115, respectively. As describe above, it is because, in the case of sitting posture, the hips usually server as stable positions. Further, the unstable posture determination unit 207 according to the present embodiment respectively determines an area 1110 including hips 1106 and 1107 and knees 1108 and 1109 as a person's body support area and an area 1120 including hips 1116 and 1117 and knees 1118 and 1119 as a person's body support area for the persons 1101 and 1102 determined to be in a sitting posture. The person's body support area according to the present embodiment is a circle with the center at a midpoint among the ankles and hips and with the diameter that is a distance obtained by adding a predetermined rate of margin to a distance between the hips and knees.

The determination method of the reference point and the person's body support area is not limited to the above-described method. For example, the reference point may be determined by including positions of other organs, such as a nose, in addition to eyes, or of other joint points. Further, the person's body support area may be determined using only the hips. The person's body support area does not necessarily have to be a circle, and may be an ellipse or a rectangle, and the shapes of the person's body support area may be different between the standing posture and sitting posture.

In step S907, the unstable posture determination unit 207 determines whether the posture of the target person is unstable based on a positional relationship between the reference point determined in step S905 and the person's body support area determined in step S906. More specifically, the unstable posture determination unit 207 determines whether the target person is in an unstable posture based on the determination result of the plurality of specific positions of the person by the skeletal frame determination unit 203 and the determination result of the gravitational direction by the gravitational direction determination unit 206. The unstable posture determination unit 207 according to the present embodiment determines whether the target person is in an unstable posture based on whether a line segment extending from the reference point in the gravitational direction reaches the person's body support area.

A case where a person is determined to be in a standing posture will be described with reference to Fig. 10. The unstable posture determination unit 207 determines that the person 1001 is in a stable posture because a line segment 1010 extending from the reference point 1005 of the person 1001 in a gravitational direction 1009 falls within the person's body support area 1008. On the other hand, the unstable posture determination unit 207 determines that the person 1002 is in an unstable posture because a line segment 1018 extending from the reference point 1013 of the person 1002 in a gravitational direction 1017 falls outside the person's body support area 1016. More specifically, the unstable posture determination unit 207 according to the present embodiment determines whether the target person is in an unstable posture based on the reference point corresponding to the positions of the target person's hips in a case where the type of posture of the person is determined to be a standing posture. Further, the unstable posture determination unit 207 determines that the target person is in an unstable posture in the case where the line segment extending from the reference point 1005 in the gravitational direction 1009 does not fall within a predetermined range (person's body support area 1008) determined based on the plurality of specific positions corresponding to the target person.

A case where the posture type determination unit 204 determines that the person is in a sitting posture will be described with reference to Fig. 11. The unstable posture determination unit 207 determines that the person 1101 is in a stable posture because a line segment 1112 extending from the reference point 1105 of the person 1101 in a gravitational direction 1111 falls within the person's body support area 1110. On the other hand, the unstable posture determination unit 207 determines that the person 1102 is in an unstable posture because a line segment 1122 extending from the reference point 1115 in a gravitational direction 1121 falls outside the person's body support area 1120. In this way, the unstable posture determination unit 207 according to the present embodiment determines whether the target person is in an unstable posture based on the reference point corresponding to the person's eye positions in the case where the posture type determination unit 204 determines that the type of posture of the person is a sitting posture. Further, the unstable posture determination unit 207 determines that the target person is in an unstable posture in the case where the line segment 1122 extending from the reference point 1115 in the gravitational direction 1121 does not fall within a predetermined range (person's body support area 1110) determined based on the plurality of specific positions corresponding to the target person.

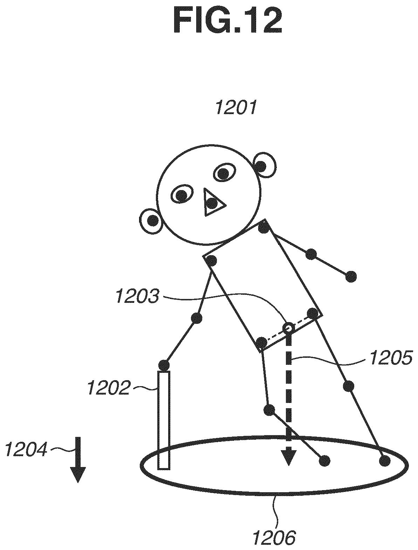

In a case where the target person is a person using a body support instrument 1202, such as a walking stick, for supporting the person's body as illustrated in Fig. 12, the unstable posture determination unit 207 detects the body support instrument 1202 and determines a person's body support area 1206 so as to include a tip position of the person's body support instrument 1202 based on a detection result. The unstable posture determination unit 207 detects the person's body support instrument 1202 using a method, for example, described in "J. Redmon, "You Only Look Once: Unified, Real-Time Object Detection", CVPR2015". The unstable posture determination unit 207 determines that a person 1201 with the body support instrument 1202 (walking stick) illustrated in Fig. 12 is in a stable posture because a line segment 1205 extending from a reference point 1203 in a gravitational direction 1204 falls within the person's body support area 1206. The posture of the person 1002 illustrated in Fig. 10 and the posture of the person 1201 illustrated in Fig. 12 are the same, but it is possible to distinguish, with higher accuracy, between the person 1002 who is actually about to fall down and the person 1201 who is not actually falling down by taking the presence of the body support instrument 1202 into consideration.

Further, as the line segment extending from the reference point in the gravitational direction is present at a position farther from the person's body support area, the posture is considered to be more unstable. Thus, the unstable posture determination unit 207 according to the present embodiment determines a degree of unstableness (unstableness degree) of each person based on a distance between the line segment and the person's body support area. For example, where a distance between a line segment extending from a reference point in a gravitational direction and a boundary of a person's body support area is "d", and a distance from the reference point to the boundary of the person's body support area is "L", an unstableness degree U can be expressed by a formula (1).

$$U = d/L \quad (1)$$

The unstableness degree U is expressed by a real number from 0 to 1. When "d" is equal to "L", the person is in a state of completely lying down, and thus "1" indicates the most unstable state. However, the unstableness degree calculation method is not limited thereto. By calculating the unstableness degree in this way, it is possible to switch the processing contents based on the unstableness degree, for example, to issuing an alert only when a person whose unstableness degree is a predetermined threshold value or more is detected. However, the unstableness degree calculation is not essential.

In step S908, the display unit 208 display a determination result by the unstable posture determination unit 207. For example, a message "There is a person in an unstable posture." may be displayed, or a rectangle surrounding a person in an unstable posture may be superimposed on a camera video image. In a case where the unstableness degree of the person's posture is calculated, a numerical value indicating the unstableness degree, or a color/bar graph or the like corresponding to the unstableness degree may be displayed. The video image processing apparatus 100 according to the present embodiment repeats the processing in Fig. 9 until an end instruction is issued by the user.

As described above, the video image processing apparatus 100 according to the present embodiment can determine whether the target person's posture is unstable from the relationship between the reference point and the person's body support area determined based on the person's specific positions. Further, the reference point and the person's body support area are set by a different method depending on the type of posture.

By using the gravity vector field described with reference to Fig. 4, it is possible to detect a person in an unstable posture accurately even if the downward direction in the screen is different from the gravitational direction due to an imaging direction or an object, such as a stool or a bench, placed in an imaging range.

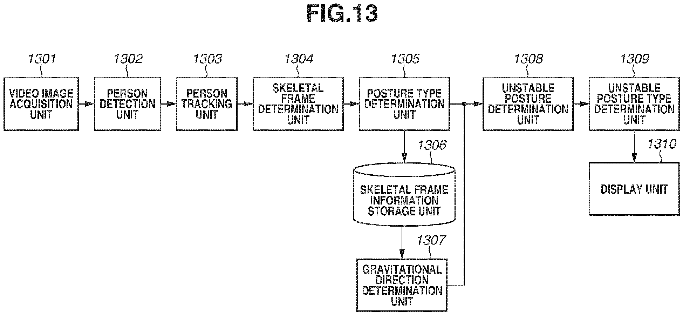

Next, a second embodiment of the present invention will be described focusing on a difference from the first embodiment. Fig. 13 is a block diagram illustrating a functional configuration of the video image processing apparatus 100 according to the second embodiment. The video image processing apparatus 100 according to the second embodiment includes a person tracking unit 1303 to be able to deal with temporal changes of a posture determination (estimation) result of a person. A video image acquisition unit 1301 and a person detection unit 1302 have functions similar to the video image acquisition unit 201 and the person detection unit 202, respectively.

The person tracking unit 1303 associates person areas acquired by the person detection unit 1302 in image frames continuous in time and assigns a same person ID. A skeletal frame determination unit 1304, a posture type determination unit 1305, and a skeletal frame information storage unit 1306 have functions similar to the skeletal frame determination unit 203, the posture type determination unit 204, and the skeletal frame information storage unit 205, respectively. Further, a gravitational direction determination unit 1307 and an unstable posture determination unit 1308 have functions similar to the gravitational direction determination unit 206 and the unstable posture determination unit 207, respectively.

An unstable posture type determination unit 1309 determines a type of unstable posture based on changes over time of output from the unstable posture determination unit 1308. A display unit 1310 has a similar function to that of the display unit 208. Further, determination processing of a gravitational direction in the second embodiment is similar to that in the first embodiment, and thus the description thereof is omitted.

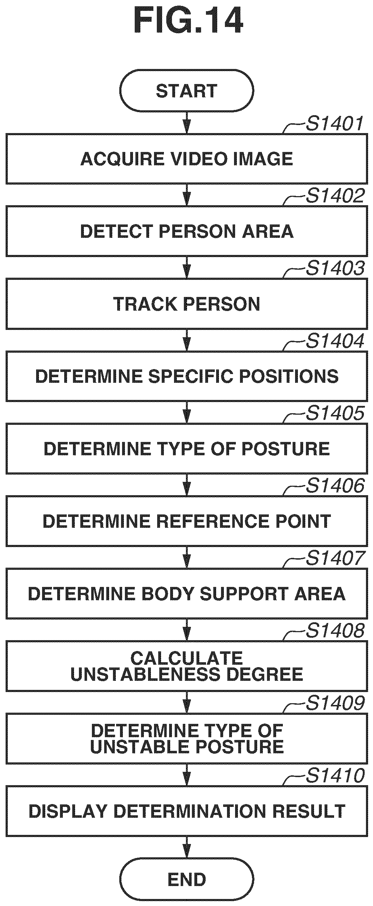

Next, details of processing performed when the video image processing apparatus 100 according to the second embodiment is in operation will be described with reference to Fig. 14. Fig. 14 is a flowchart illustrating processing executed by the video image processing apparatus 100 according to the present embodiment to determine (estimate) a person's posture. The processing in each step illustrated in Fig. 14 is implemented by the CPU 101 of the video image processing apparatus 100 reading a necessary program from the ROM 102 into the RAM 103 and executing the program. Further, the processing in Fig. 14 starts when a user issues an instruction to execute a posture determination. However, for example, the processing in Fig. 14 may be started automatically when a predetermined condition is satisfied. Processing performed in steps S1401 and S1402 is similar to the processing performed in steps S901 and S902, respectively, and thus the description thereof is omitted.

In step S 1403, the person tracking unit 1303 determines whether the person area detected in a current frame corresponds to one or a plurality of person areas detected in a previous frame. The person tracking unit 1303 assigns a same person ID to the person areas determined to include the same person.

There are various methods of performing tracking processing, and, for example, there is a method of associating the person areas in the frames in which a distance between a center position of the person area included in the previous frame and a center position of the person area included in the current frame is the shortest. Other than the above-described method, there are various methods of associating the person areas in the frames, such as a pattern matching method using the person area in the previous frame as a collation pattern.

Processing performed in steps S 1404, S 1405, S 1406, and S 1407 is similar to the processing performed in steps S903, S904, S905, and S906, respectively, and thus the description thereof is omitted.

In step S1408, the unstable posture determination unit 1308 calculates a person's unstableness degree using a method similar to that in step S907. Further, the unstable posture determination unit 1308 temporarily stores, in the RAM 103, an unstableness degree information list in which unstableness degrees are time-sequentially arranged for each person (person ID). In step S 1409, the unstable posture type determination unit 1309 reads the unstableness degree information list temporarily stored in the RAM 103, and determines the type of unstable posture for each person ID.

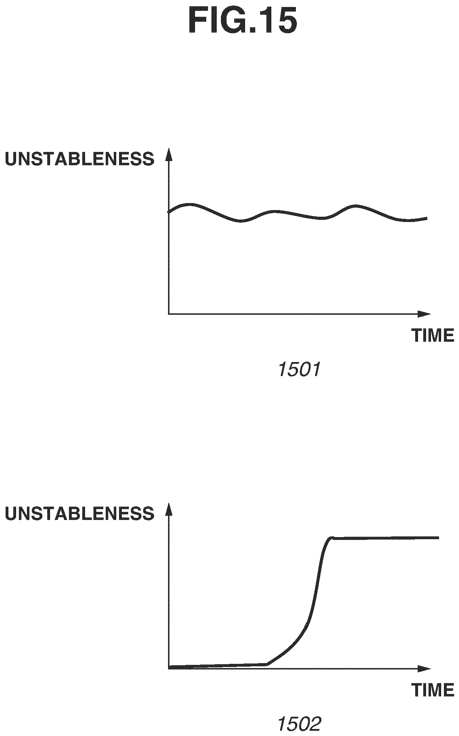

Fig. 15 illustrates examples of graphs each with horizontal and vertical axes representing time and unstableness, respectively. A graph 1501 illustrates a state where the unstableness degree continues to be at a predetermined level or more. Such a state is considered to indicate a "swaying" action. In contrast, a graph 1502 illustrates a state where the unstableness degree increases rapidly. Such a state is considered to indicate a "falling down" action. However, the action determination method is not limited thereto, and the actions determined based on temporal changes of the unstableness degree are not limited to "swaying" and "falling down".

In step S1410, the display unit 1310 displays a determination result of the unstable posture. For example, a message "There is a swaying person." may be displayed, or a rectangle with a color corresponding to the type of unstable posture and surrounding the person may be superimposed on a camera video image. The video image processing apparatus 100 according to the present embodiment repeats the processing in Fig. 14 until an end instruction is issued by the user. As described above, the video image processing apparatus 100 according to the present embodiment can determine the type of unstable posture based on the temporal changes of the unstableness degree.

<Other Embodiments>

In the above-described embodiments, the description is given of the case where the display unit 1310 displays the determination result by the unstable posture determination unit 207. In addition, a video image captured when an unstable person is detected may be recorded, or meta-information indicating the time when the unstable person is detected may be added. In this way, the video image captured when the unstable person is detected can be easily searched for.

Further, in the above-described embodiments, the description is given focusing on the examples in which the person's body support area is determined based on the plurality of specific positions of the person, but it is not limited thereto. For example, an area obtained by adding a predetermined margin to one of the person's ankles may be the person's body support area. In this way, even in a case where a person has turned sideways relative to the camera and only one of the person's legs is visible, it is possible to determine whether the person is in an unstable posture.

Further, in the above-described embodiments, the description is given of the examples in which the video image processing apparatus 100 has all the functions illustrated in Fig. 2 or Fig. 13, but it is not limited thereto. For example, the processing performed by the person detection unit 202 to the processing performed by the unstable posture determination unit 207 may be performed on the cloud, and a result thereof may be displayed on a display installed in a store or the like. According to the present invention, it is possible to distinguish person's actions accurately.

Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to embodiments, it is to be understood that the invention is not limited to the disclosed embodiments, but is defined by the scope of the following claims.

**Claims**

1. A video image processing apparatus comprising:

   acquisition means (201) configured to acquire a video image;
   position determination means (202, 203) configured to determine a plurality of specific positions of a person (502, 602, 603, 1001, 1002, 1101, 1102, 1201) in the video image acquired by the acquisition means (201);
   gravitational direction determination means (206) configured to determine a gravitational direction (1009, 1017, 1111, 1121, 1204) in at least a part of an area in the video image acquired by the acquisition means (201); and posture determination means (204, 207) configured to determine whether the person (502, 602, 603, 1001, 1002, 1101, 1102, 1201) is in an unstable posture based on a determination result of the plurality of specific positions of the person (502, 602, 603, 1001, 1002, 1101, 1102, 1201) by the position determination means (202, 203) and a determination result of the gravitational direction (1009, 1017, 1111, 1121, 1204) by the gravitational direction determination means (206).

2.  The apparatus according to claim 1, wherein the plurality of specific positions includes at least a position regarding one or more of a joint (301-312, 605-606, 608-609, 615, 616), a head, an eye (313, 314, 612, 613), a nose (317), an ear (315, 316) and a mouth of the person (502, 602, 603, 1001, 1002, 1101, 1102, 1201).

3.  The apparatus according to claim 1 or 2,

    wherein a reference point (1005, 1013, 1105, 1115) of the person (502, 602, 603, 1001, 1002, 1101, 1102, 1201) and a body support area (1008, 1016, 1110, 1120, 1206) of the person (502, 602, 603, 1001, 1002, 1101, 1102, 1201) are identified based on the plurality of specific positions of the person (502, 602, 603, 1001, 1002, 1101, 1102, 1201), and
    wherein whether the person (502, 602, 603, 1001, 1002, 1101, 1102, 1201) is in an unstable posture is determined based on a positional relationship between the reference point (1005, 1013, 1105, 1115) and the body support area (1008, 1016, 1110, 1120, 1206).

4.  The apparatus according to claim 3, wherein the person (502, 602, 603, 1001, 1002, 1101, 1102, 1201) is determined to be in an unstable posture in a case where a line segment extending from the reference point (1005, 1013, 1105, 1115) of the person (502, 602, 603, 1001, 1002, 1101, 1102, 1201) in the gravitational direction (1009, 1017, 1111, 1121, 1204) does not fall within a predetermined range determined based on the plurality of specific positions corresponding to the person (502, 602, 603, 1001, 1002, 1101, 1102, 1201).

5.  The apparatus according to claim 3,

    wherein a type of posture of the person (502, 602, 603, 1001, 1002, 1101, 1102, 1201) is determined based on the plurality of specific positions, and
wherein whether the person (502, 602, 603, 1001, 1002, 1101, 1102, 1201) is in an unstable posture is determined based on the reference point (1005, 1013, 1105, 1115) corresponding to the determined type of posture.

6.  The apparatus according to claim 5,

    wherein, in a case where the type of posture of the person (502, 602, 603, 1001, 1002, 1101, 1102, 1201) is determined to be a standing posture, whether the person (502, 602, 603, 1001, 1002, 1101, 1102, 1201) is in an unstable posture is determined based on the reference point (1005, 1013, 1105, 1115) corresponding to a position of a hip (1003, 1004, 1011, 1012, 1106, 1107, 1116, 1117) of the person (502, 602, 603, 1001, 1002, 1101, 1102, 1201), and
    wherein, in a case where the type of posture of the person (502, 602, 603, 1001, 1002, 1101, 1102, 1201) is determined to be a sitting posture, whether the person (502, 602, 603, 1001, 1002, 1101, 1102, 1201) is in an unstable posture is determined based on the reference point (1005, 1013, 1105, 1115) corresponding to a position of an eye (313, 314, 612, 613, 1103, 1104, 1113, 1114) of the person (502, 602, 603, 1001, 1002, 1101, 1102, 1201).

7.  The apparatus according to any one of the preceding claims, wherein the gravitational direction (1009, 1017, 1111, 1121, 1204) is determined for each of one or more areas in the video image.

8.  The apparatus according to any one of the preceding claims, wherein an unstableness degree of the person (502, 602, 603, 1001, 1002, 1101, 1102, 1201) is further determined based on the determination result of the plurality of specific positions and the determination result of the gravitational direction (1009, 1017, 1111, 1121, 1204).

9.  The apparatus according to claim 8, wherein a type of the unstable posture is further determined based on a temporal change of the unstableness degree of the person (502, 602, 603, 1001, 1002, 1101, 1102, 1201).

10. The apparatus according to any one of the preceding claims, further comprising detection means (207) configured to detect a body support instrument (1202) from the video image, wherein whether the person (502, 602, 603, 1001, 1002, 1101, 1102, 1201) is in an unstable posture is determined based on the determination result of the plurality of specific positions of the person (502, 602, 603, 1001, 1002, 1101, 1102, 1201), the determination result of the gravitational direction (1009, 1017, 1111, 1121, 1204), and a detection result of the body support instrument (1202).

11. The apparatus according to any one of the preceding claims, wherein the gravitational direction (1009, 1017, 1111, 1121, 1204) is determined based on a reference point (1005, 1013, 1105, 1115) corresponding to a posture of the person (502, 602, 603, 1001, 1002, 1101, 1102, 1201) in the video image.

12. A posture determination method comprising:

acquiring (S401, S901, S1401) a video image;
determining (S403, S903, S1404) a plurality of specific positions of a person (502, 602, 603, 1001, 1002, 1101, 1102, 1201) in the acquired video image;
determining (S408, S409) a gravitational direction (1009, 1017, 1111, 1121, 1204) in at least a part of an area in the acquired video image; and
determining (S907, S1409) whether the person (502, 602, 603, 1001, 1002, 1101, 1102, 1201) is in an unstable posture based on a determination result of the plurality of specific positions of the person (502, 602, 603, 1001, 1002, 1101, 1102, 1201) and a determination result of the gravitational direction (1009, 1017, 1111, 1121, 1204).

13. The method according to claim 12, wherein a reference point (1005, 1013, 1105, 1115) of the person (502, 602, 603, 1001, 1002, 1101, 1102, 1201) and a body support area (1008, 1016, 1110, 1120, 1206) of the person (502, 602, 603, 1001, 1002, 1101, 1102, 1201) are identified based on the plurality of specific positions of the person (502, 602, 603, 1001, 1002, 1101, 1102, 1201), and whether the person (502, 602, 603, 1001, 1002, 1101, 1102, 1201) is in an unstable posture is determined based on a positional relationship between the reference point (1005, 1013, 1105, 1115) and the body support area (1008, 1016, 1110, 1120, 1206).

14. The method according to claim 13, wherein the person (502, 602, 603, 1001, 1002, 1101, 1102, 1201) is determined to be in an unstable posture in a case where a line segment extending from the reference point (1005, 1013, 1105, 1115) of the person (502, 602, 603, 1001, 1002, 1101, 1102, 1201) in the gravitational direction (1009, 1017, 1111, 1121, 1204) does not fall within a predetermined range determined based on the plurality of specific positions corresponding to the person (502, 602, 603, 1001, 1002, 1101, 1102, 1201).

15. A computer program comprising instructions which, when the program is executed by a computer, cause the computer to carry out the method according to any one of claims 12 to 14.

# FIG.1

*100*

*101* CPU

*102* ROM

*103* RAM

*104* SECONDARY STORAGE DEVICE

*109* BUS

*105* IMAGING DEVICE

*106* INPUT DEVICE

*107* DISPLAY DEVICE

*108* NETWORK I/F

# FIG.2

| 201 | 202 | 203 | 204 | | 207 | 208 |
|---|---|---|---|---|---|---|
| VIDEO IMAGE ACQUISITION UNIT | PERSON DETECTION UNIT | SKELETAL FRAME DETERMINATION UNIT | POSTURE TYPE DETERMINATION UNIT | | UNSTABLE POSTURE DETERMINATION UNIT | DISPLAY UNIT |

205

SKELETAL FRAME INFORMATION STORAGE UNIT

206

GRAVITATIONAL DIRECTION DETERMINATION UNIT

# FIG.3

# FIG.4

```
                    ┌─────────────┐
                    │    START    │
                    └─────────────┘
                           │
                           ▼              ┌─S401
              ┌─────────────────────────┐
              │   ACQUIRE VIDEO IMAGE    │
              └─────────────────────────┘
                           │
                           ▼              ┌─S402
              ┌─────────────────────────┐
              │    DETECT PERSON AREA    │
              └─────────────────────────┘
                           │
                           ▼              ┌─S403
              ┌─────────────────────────┐
              │ DETERMINE SPECIFIC       │
              │        POSITIONS         │
              └─────────────────────────┘
                           │
                           ▼              ┌─S404
              ┌─────────────────────────┐
              │ DETERMINE TYPE OF POSTURE│
              └─────────────────────────┘
                           │
                           ▼              ┌─S405
              ┌─────────────────────────┐
              │     STORE SKELETAL       │
              │    FRAME INFORMATION     │
              └─────────────────────────┘
                           │
                           ▼                    S406
                  ◇─────────────────────◇
          NO      │  HAS PREDETERMINED  │
      ◄───────────┤   TIME ELAPSED?     │
                  ◇─────────────────────◇
                           │
                        YES ▼            ┌─S407
              ┌─────────────────────────┐
              │     READ SKELETAL        │
              │    FRAME INFORMATION     │
              └─────────────────────────┘
                           │
                           ▼              ┌─S408
              ┌─────────────────────────┐
              │    SELECT DIRECTION      │
              │    USED TO DETERMINE     │
              │  GRAVITATIONAL DIRECTION │
              └─────────────────────────┘
                           │
                           ▼              ┌─S409
              ┌─────────────────────────┐
              │ DETERMINE GRAVITY VECTOR │
              │          FIELD           │
              └─────────────────────────┘
                           │
                           ▼
                    ┌─────────────┐
                    │     END     │
                    └─────────────┘
```

# FIG.5

# FIG.6

EP 4 481 695 A1

# FIG.7

18

# FIG.8

# FIG.9

START

ACQUIRE VIDEO IMAGE　—S901

DETECT PERSON AREA　—S902

DETERMINE SPECIFIC POSITIONS　—S903

DETERMINE TYPE OF POSTURE　—S904

DETERMINE REFERENCE POINT　—S905

DETERMINE BODY SUPPORT AREA　—S906

DETERMINE UNSTABLE POSTURE　—S907

DISPLAY DETERMINATION RESULT　—S908

END

# FIG.10

# FIG.11

# FIG.12

# FIG.13

```
   1301           1302          1303          1304              1305                      1308              1309
┌──────────┐  ┌──────────┐  ┌──────────┐  ┌──────────┐  ┌──────────────┐      ┌──────────────┐  ┌──────────────┐
│VIDEO IMAGE│→│  PERSON  │→│  PERSON  │→│ SKELETAL │→│ POSTURE TYPE │→·····→│   UNSTABLE   │→│   UNSTABLE   │
│ACQUISITION│ │DETECTION │ │ TRACKING │ │  FRAME   │ │DETERMINATION │      │   POSTURE    │ │POSTURE TYPE  │
│   UNIT    │ │   UNIT   │ │   UNIT   │ │DETERMINATION│ │    UNIT    │      │DETERMINATION │ │DETERMINATION │
└──────────┘  └──────────┘  └──────────┘  │   UNIT   │ └──────────────┘      │    UNIT      │ │    UNIT      │
                                          └──────────┘         │            └──────────────┘  └──────────────┘
                                                               ↓                                      │
                                                            1306                                   1310
                                                     ┌──────────────┐                          ┌──────────────┐
                                                     │SKELETAL FRAME│                          │ DISPLAY UNIT │
                                                     │ INFORMATION  │                          └──────────────┘
                                                     │ STORAGE UNIT │
                                                     └──────────────┘
                                                            │
                                                            ↓  1307
                                                     ┌──────────────┐
                                                     │GRAVITATIONAL │
                                                     │  DIRECTION   │
                                                     │DETERMINATION │
                                                     │    UNIT      │
                                                     └──────────────┘
```

# FIG.14

```
        ┌─────────────┐
        │    START    │
        └──────┬──────┘
               │           ╭─S1401
        ┌──────▼──────────────┐
        │  ACQUIRE VIDEO IMAGE │
        └──────┬──────────────┘
               │           ╭─S1402
        ┌──────▼──────────────┐
        │  DETECT PERSON AREA  │
        └──────┬──────────────┘
               │           ╭─S1403
        ┌──────▼──────────────┐
        │    TRACK PERSON      │
        └──────┬──────────────┘
               │           ╭─S1404
        ┌──────▼──────────────────┐
        │ DETERMINE SPECIFIC POSITIONS │
        └──────┬──────────────────┘
               │           ╭─S1405
        ┌──────▼──────────────────┐
        │ DETERMINE TYPE OF POSTURE │
        └──────┬──────────────────┘
               │           ╭─S1406
        ┌──────▼──────────────────┐
        │ DETERMINE REFERENCE POINT │
        └──────┬──────────────────┘
               │           ╭─S1407
        ┌──────▼──────────────────┐
        │ DETERMINE BODY SUPPORT AREA │
        └──────┬──────────────────┘
               │           ╭─S1408
        ┌──────▼──────────────────┐
        │      CALCULATE           │
        │  UNSTABLENESS DEGREE     │
        └──────┬──────────────────┘
               │           ╭─S1409
        ┌──────▼──────────────────┐
        │  DETERMINE TYPE OF       │
        │  UNSTABLE POSTURE        │
        └──────┬──────────────────┘
               │           ╭─S1410
        ┌──────▼──────────────────┐
        │ DISPLAY DETERMINATION RESULT │
        └──────┬──────────────────┘
               │
        ┌──────▼──────┐
        │     END     │
        └─────────────┘
```

25

# FIG.15

UNSTABLENESS

TIME

*1501*

UNSTABLENESS

TIME

*1502*

Europäisches
Patentamt

European
Patent Office

Office européen
des brevets

# EUROPEAN SEARCH REPORT

Application Number

EP 24 18 2106

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/331028 A1 (STERNITZKE CHRISTIAN [DE] ET AL) 20 October 2022 (2022-10-20) * paragraphs [0046], [0315] - [0321], [0325], [0340] - [0343], [0350] - [0354], [0384] * ----- | 1-15 | INV. G06V10/764 A61B5/11 G06T7/73 G06V40/10 G06V40/20 G08B21/04 |
| X | US 2018/068179 A1 (DERENNE RICHARD A [US] ET AL) 8 March 2018 (2018-03-08) * paragraphs [0205] - [0206] * ----- | 1,12,15 | |

TECHNICAL FIELDS SEARCHED (IPC)

G06V
G08B
A61B
G06T

The present search report has been drawn up for all claims

| Place of search | Date of completion of the search | Examiner |
|---|---|---|
| The Hague | 14 October 2024 | Mukasa, Oliver |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   ...............................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 18 2106

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2024

| Patent document cited in search report | Publication date | Patent family member(s) | | Publication date |
|---|---|---|---|---|
| US 2022331028 A1 | 20-10-2022 | CN | 114980810 A | 30-08-2022 |
| | | EP | 4003164 A1 | 01-06-2022 |
| | | US | 2022331028 A1 | 20-10-2022 |
| | | WO | 2021038109 A1 | 04-03-2021 |
| US 2018068179 A1 | 08-03-2018 | US | 2015109442 A1 | 23-04-2015 |
| | | US | 2018068179 A1 | 08-03-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022021940 A

**Non-patent literature cited in the description**

- **RENE RANFTL et al.** Vision Transformers for Dense Prediction. *arXiv: 2103.13413*

- **J. REDMON**. You Only Look Once: Unified, Real-Time Object Detection. *CVPR2015*